Europäisches Patentamt

European Patent Office

Office européen des brevets

(11) Publication number: **0 196 185**
**B1**

# EUROPEAN PATENT SPECIFICATION

(45) Date of publication of patent specification: **12.07.89**

(51) Int. Cl.⁴: **A 61 K 31/70, C 07 H 19/073**

(21) Application number: **86301897.4**

(22) Date of filing: **14.03.86**

(60) Divisional applications 88101790, 88101795 filed on 08.02.88.

(54) Antiviral nucleosides.

(30) Priority: 16.03.85 GB 8506869
09.05.85 GB 8511774
27.09.85 GB 8523881
12.02.86 GB 8603450
17.09.85 US 776899

(43) Date of publication of application:
01.10.86 Bulletin 86/40

(45) Publication of the grant of the patent:
12.07.89 Bulletin 89/28

(84) Designated Contracting States:
AT BE CH DE FR GB IT LI LU NL SE

(56) References cited:
EXPERIMENTAL CELL RESEARCH, vol. 116, no.
1, 1978, Academic Press Inc., SE, pp. 31-37, W.
OSTERTAG et al.: "Induction of intracisternal
A-type particles during friend cell
differentiation"
pp. 21-29, C.J. KRIEG et al.: "Increase in
intracisternal A-type particles in Friend cells
during inhibition of Friend virus (SFFV) release
by interferon or azidothymidine"

(73) Proprietor: THE WELLCOME FOUNDATION
LIMITED
183-193 Euston Road
London NW1 2BP (GB)

(72) Inventor: Rideout, Janet Litster
3101 Morningside Drive
Raleigh North Carolina (US)
Inventor: Barry, David Walter
1810S Lakeshore Drive
Chapel Hill North Carolina (US)
Inventor: Lehrman, Sandra Nusinoff
2720 Old Sugar Road
Durham North Carolina (US)
Inventor: St. Clair, Martha Heider
312 Seven Oaks Road
Durham North Carolina (US)
Inventor: Furman, Phillip Allen
901 Bluestone Road
Durham North Carolina (US)

(74) Representative: Garrett, Michael et al
The Wellcome Foundation Limited Group
Patents and Agreements Langley Court
Beckenham Kent BR3 3BS (GB)

(56) References cited:

PROC. NATL. ACAD. SCI. USA, vol. 71, no. 12, December 1974, pp. 4980-4985, US, W. OSTERTAG et al.: "Induction of endogenous virus and of thymidine kinase by bromodeoxyuridine in cell cultures transformed by friend-virus"

PROC. NATL. ACAD. SCI. USA, vol. 82, October 1985, pp. 7096-7100, US, H. MITSUYA et al.: "3'-Azido-3'-deoxythymidine (BW A509U): An antiviral agent that inhibits the infectivity and cytopathic effect of human T-lymphotropic virus type III/lymphadenopathy-associated virus in vitro"

DIALOG INFORMATION SERVICES, file 267; DeHaen Drug Data, 1980-1987, ref.no. 0180178: M.H. St Clair et al.: "Characterization of HTLV-3 reverse transciotase and inhibition by the triphosphate of BW A509U"

REf.no. 0180181: W.D. HARDY Jr. et al.: "Anti-viral effects of BW A509U against a naturally occuring feline acquired immune deficiency syndrome (FAIDS)-inducing retrovirus"

Ref.No. 0180180: H. MITSUYA et al.: "BW A509U blocks HTLV-3 infection of T-lymphocytes in culture"

Ref.No. 0180179: P.A. FURMAN et al.: "Selective inhibition of HTLV-3 by BW A509U." JOURNAL OF THE AM. MED. ASSOC., vol. 254, no. 18, November 8, 1985, pp. 2521,2527,2529, D.E. RIESENBERG: "Anti-AIDS agents show varying early results in vitro and in vivo"

DIALOG INFORMATION SERVICES, file 5; Biosis Previews 69-87/June, ref.no. 0016194545: W.D. HARDY Jr. et al.: "Azidothymidine inhibits the replication of the feline leukemia virus"

Chem. & ENG. NEWS, vol. 64, no. 4, January 27, 1986, pp. 28-40, R.K. ROBINS: "Synthetic antiviral agents"

FDA DRUG BULLETIN, vol. 15, no. 3, October 1985, pp. 27-32, NN: "Progress on AIDS" PROCEEDINGS OF AM. ASSOC. CANCER RES., vol. 27, 77th Meeting, March 1986, p. 422, ref. no. 1675, R.M. RUPRECHT et al.: "3'-Azido-3'-deoxythymidine (AZT) as antiretroviral agent in murine systems in vitro and in vivo"

PROCEEDINGS OF AM. SOC. CLIN. ONCOL., vol. 5, 22nd Meeting, March 1986, p. 1, ref.no. 4, R.W. KLECKER et al.: "Clinical pharmacology of 3'-azido-3'-deoxythymidine (N3TdR), a novel pyrimidine analog with potential application for the treatment of patients with AIDS and related diseases"

CANCER RESEARCH, vol. 32, no. 7, July 1972, pp. 1547-1553, A.W. SCHRECKER et al.: "Inhibition of RNA-dependent DNA polymerase by thymidylate derivatives"

NATURE, vol. 323, October 2, 1986, pp. 467-469, R.M. RUPRECHT et al.: "Suppression of mouse viraemia and retroviral disease by 3'-azido-3'-deoxythymidine"

THE LANCET, vol. I, no. 8539, April 25, 1987, pp. 957-958, NN: "Zidovudine (AZT)"

**Description**

The invention relates to pharmaceutical formulations containing 3'-azido-3'-deoxythymidine for use in the treatment or prophylaxis of human retroviral infections.

Retroviruses form a sub-group of RNA viruses which, in order to replicate, must first 'reverse transcibe' the RNA of their genome into DNA ('transcription' conventionally describes the synthesis of RNA from DNA). Once in the form of DNA, the viral genome is incorporated into the host cell genome, allowing it to take full advantage of the host cell's transcription/translation machinery for the purposes of replication. Once incorporated, the viral DNA is virtually indistinguishable from the host's DNA and, in this state, the virus may persist for as long as the cell lives. As it is virtually invulnerable to attack in this form, any treatment must be directed at another state of the life cycle and will, of necessity, have to be continued until all virus-carrying cells have died.

A species of retrovirus has now been reproducibly isolated form patients with AIDS (Acquired Immune Deficency Syndrome). While it has been extensively characterised, there is, as yet, no agreed name for the virus, and it is currently known either as human T-cell lymphotropic virus III (HTLV III). AIDS-associated retrovirus (ARV). or lymphadenopathy-associated virus (LAV). It is anticipated that the name to be agreed on internationally is Acquired Immune Deficiency Virus (AIDV). This virus (referred to herein as AIDV) has been shown preferentially to infect and destroy T-cells bearing the OKT[4] surface marker and is now generally accepted as the aetiologic agent of AIDS. The patient progressively loses this set of T-cells, upsetting the overall balance of the immune system, reducing his ability to combat other infections, and predisposing him to opportunistic infections which frequently prove fatal. Thus, the usual cause of death in AIDS victims is by opportunistic infection, such as pneumonia or virally induced cancers, and not as a direct result of AIDV infection.

Recently, AIDV has also been recovered from other tissue types, including B- cells expressing the T[4] marker, macrophages and non-blood associated tissue in the central nervous system (CNS). This latter infection has been discovered in patients expressing classical AIDS symptoms and is associated with progressive demyelination, leading to wasting and such symptoms as encephalopathy, progressive dysarthria, ataxia and disorientation.

There are at least four clinical manifestations of AIDV infection. In the initial 'carrier' state, the only indication of infection is the presence of anti-AIDV antibodies in the blood-stream. It is believed that such 'carriers' are capable of passing on the infection, e.g. by blood transfusion, sexual intercourse, or used syringe needles. The carrier state may often never progress to the second stage characterised by persistent generalised lymphadenopathy (PGL). It is currently estimated that about 20% of PGL patients progress to a more advanced condition known as 'AIDS related complex' (ARC). Physical symptoms associated with ARC may include general malaise, increased temperature and chronic infections. This condition usually progresses to the final, fatal AIDS condition, when the patient completely loses the ability to fight infection.

The existence of these human retroviruses and others has only recently been recognised and, as the diseases with which they are linked are of a life-threatening nature, there exists an urgent need to develop ways to combat these virsues.

Various drugs have now been proposed as 'cures' for AIDS. These include antimoniotungstate, suramin, ribavirin and isoprinosine, which are either somewhat toxic or have shown no marked anti-retroviral activity. As the AIDV genome is incorporated into the host cell DNA after infection and is virtually invulnerable to attack in this state, it will persist as long as the host cell survives, causing new infection in the meantime. Thus, any treatment of AIDS would have to be for an extended period, possibly life, requiring substances with an acceptable toxicity.

Reports have described the testing of compounds against retroviruses, for example, Friend Leukaemia Virus (FLV), a murine retrovirus. For instance Krieg *et al.* (Exp. Cell Res., *116* (1978) 21—29) found that 3'-azido-3'-deoxythymidine affected the release of C-type particles and increased the formation of A-type particles of a FLV complex in *in vitro* experiments, and Ostertag *et al.* (Proc. Nat. Acad. Sci. (1974) *71,* 4980—85) stated that, on the basis of antiviral activity related to the above FLV complex and a lack of cellular toxicity, 3'-azido-3'-deoxythymidine "might favourably replace bromodeoxyuridine for medical treatment of diseases caused by DNA viruses". However, De Clerq *et al.* (Biochem. Pharm. (1980) *29,* 1849—1851) established, six years later, that 3'-azido-3'-deoxythymidine had no appreciable activity against any viruses used in their tests, including vaccinia, HSVI and varicella zoster virus (VZV).

We have now discovered that 3'-azido-3'-deoxythymidine has a surprisingly potent activity against human retroviruses, with a particularly high activity against AIDV as demonstrated by the experimental data referred to below. Such activity renders the compound useful in the therapy of human retroviral infections.

According to the present invention, we provide pharmaceutical formulations comprising, as active ingredient, 3'-azido-3'-deoxythymidine and at least one pharmatuceutically acceptable carrier therefor.

Activity of 3'-azido-3'-deoxythymidine against human retroviruses has been established in various *in vitro* assay systems. For example, infection of the H9 human lymphoblastoid cell-line by AIDV is effectively prevented by concentrations of 3'-azido-3'-deoxythymidine as low as 0.013 mcg/ml up to 20 hours after infection. AIDV infection of U937 human lymphoblastoid cells, PHA- stimulated white blood cells and cultured peripheral blood lymphocytes is also prevented at similarly low concentrations. In addition, 10-day challenge experiments using up to 5000 AIDV virions per cell and cloned T4, tetanus- specific, T-helper lymphocytes, showed no decrease in cells treated with 3'-azido-3'-deoxythymidine, while untreated cells had decreased 5-fold. Cytopathic effects were also completely blocked in the same cell-line transformed by HTLV-I and super-infected with AIDV.

Other studies using purified AIDV reverse transcriptase have shown that the activity of this enzyme is blocked by the triphosphate of 3'-azido-3'-deoxythymidine by a competitive inhibition mechanism.

Phase I clinical trials have also shown that 3'-azido-3'-deoxythymidine is capable of crossing the blood/brain barrier in clinically effective quantities. This property is both unusual and valuable for the treatment and prophylaxis of CNS infections caused by human retroviruses.

The ability of 3'-azido-3'-deoxythymidine to modify the course of retrovirus induced malignancy has been demonstrated in a mouse model, whereby administration of 3'-azido-3'-deoxythymidine prevented splenomegaly caused by intravenously administered Rauscher Murine Leukaemia Virus, the murine equivalent of HTLV-I. In further experiments, 3'-azido-3'-deoxythymidine has been shown to inhibit the *in vitro* replication of HTLV-I at concentrations as low as 0.8 mcg/ml.

Experiments have shown that 3'-azido-3'-deoxythymidine is converted, *in vivo*, by the action of cellular enzymes into the 5'-monophosphate. The monophosphate is then further phosphorylated by other enzymes to form the triphosphate *via* the diphosphate, and other studies have demonstrated that it is the triphosphate form of 3'-azido-3'-deoxythymidine which is believed to be the effective chain terminator in the reverse transciprtion of AIDV, as evidenced by its effect on avian myeloblastosis virus and Moloney murine leukaemia virus. This form also inhibits AIDV reverse transcriptase *in vitro* whilst having a negligible effect on human DNA polymerase activity.

Examples of human retrovirus infections which may be treated or prevented by 3'-azido-3'-deoxythymidine using the pharmaceutical formulations according to the invention include T-cell lymphotropic retroviruses (HTLV), especially HTLV-I, HTLV-II and AIDV (HTLV-III). Clinical conditions that may be treated or prevented in accordance with the invention include AIDS, AIDS-related complex and HTLV-I positive leukaemia and lymphoma. Suitable patients for treatment also include those having antibodies to AIDV, AIDV CNS infections, PGL and ARC.

3'-azido-3'-deoxythymidine (hereafter referred to as the active ingredient) may be administered to humans for prophylaxis or treatment of retroviral infections by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient and the nature of the infection.

In general a suitable dose will be in the range of 3.0 to 120 mg per kilogram body weight of the patient per day, preferably in the range of 6 to 90 mg per kilogram body weight per day and most preferably in the range 15 to 60 mg per kilogram body weight per day. The desired dose is preferbaly presented as two, three, four, five, six or more sub-doses administered at appropriate intervals throughout the day. These sub-doses may be administered in unit dosage forms, for example, containing 5 to 1500 mg, preferably 10 to 1000 mg, and most preferably 20 to 700 mg of active ingredient per unit dosage form.

Experiments with 3'-azido-3'-deoxythymidine suggest that a dose should be administered to achieve peak plasma concentrations of the active compound of from about 1 to about 75 μM, preferably about 2 to 50 μM, most preferably about 3 to about 30 μM. This may be achieved, for example, by the intravenous injection of a 0.1 to 5% solution of the active ingredient, optionally in saline, or orally administered as a bolus containing about 1 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

The pharmaceutical formulations according to the present invention may optionally include one or more further therapeutic agents. The pharmaceutically acceptable carrier referred to above must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste. Oral formulations may further include other agents conventional in the art, such as sweeteners, flavouring agents and thickeners. Pharmaceutical formulations according to the invention include those providing sustained release of the active ingredient after oral administration.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluant, preservative, disintegrant (e.g. sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of an active ingredient.

3'-Azido-3'-deoxythymidine may also be used in therapy in conjunction with other medicaments such as 9-[(2-hydroxy-1-(hydroxy-methyl)ethoxy]methyl]guanine, 9-(2-hydroxyethoxymethyl)guanine (acyclovir), 2-amino-9-(2-hydroxyethoxymethyl)purine, interferon, e.g., α-interferon, interleukin II, and phosphonoformate (Foscarnet) or in conjunction with other immune modulating therapy including bone marrow or lymphocyte transplants or medications such as levamisol or thymosin which serve to increase lymphocyte numbers and/or function as appropriate.

It should be understood that in addition to the ingredients particularly mentioned above the formulations of this invention may include other agents conventional in the art of formulation.

3'-Azido-3'-deoxythymidine may be prepared in conventional manner, for example as described in the following references, or by methods analogous thereto: J. R. Horwitz et al., J. Org. Chem. 29, (July 1964) 2076—78; M. Imazawa et al., J. Org. Chem. 43 (15) (1978) 3044—3048; K. A. Watanabe et al., J. Org. Chem., 45, 3274 (1980); and R. P. Glinski et al., J. Chem. Soc. Chem. Commun., 915 (1970), as well as the process described in the Reference Example hereinafter.

The following Examples are intended for illustration only and are not intended to limit the scope of the invention in any way. The term 'active ingredient' as used in the Examples means 3'-azido-3'-deoxythymidine.

Example 1: Tablet formulations:

The following formulations A to C were prepared by wet granulation of the ingredients with a solution of povidone, followed by addition of magnesium stearate and compression.

| Formulation A | mg/tablet | mg/tablet |
|---|---|---|
| (a) Active ingredient | 250 | 250 |
| (b) Lactose B.P. | 210 | 26 |
| (c) Povidone B.P. | 15 | 9 |
| (d) Sodium Starch Glycollate | 20 | 12 |
| (e) Magnesium Stearate | 5 | 3 |
| | 500 | 300 |

## EP 0 196 185 B1

| Formulation B | mg/tablet | mg/tablet |
|---|---|---|
| (a) Active ingredient | 250 | 250 |
| (b) Lactose | 150 | — |
| (c) Avicel PH 101 | 60 | 26 |
| (d) Povidone B.P. | 15 | 9 |
| (e) Sodium Starch Glycollate | 20 | 12 |
| (f) Magnesium Stearate | 5 | 3 |
| | 500 | 300 |

| Formulation C | mg/tablet |
|---|---|
| Active ingredient | 100 |
| Lactose | 200 |
| Starch | 50 |
| Povidone | 5 |
| Magnesium stearate | 4 |
| | 359 |

The following formulations, D and E, were prepared by direct compression of the admixed ingredients. The lactose used in formulation E was of the direct compression type (Dairy Crest-"Zeparox").

| Formulation D | mg/tablet |
|---|---|
| Active Ingredient | 250 |
| Pregelatinised Starch NF15 | 150 |
| | 400 |

| Formulation E | mg/tablet |
|---|---|
| Active ingredient | 250 |
| Lactose | 150 |
| Avicel | 100 |
| | 500 |

Formulation F (controlled release formulation)

The formulation was prepared by wet granulation of the ingredients (below) with a solution of povidone followed by the addition of magnesium stearate and compression.

| | mg/tablet |
|---|---|
| (a) Active ingredient | 500 |
| (b) Hydroxypropylmethylcellulose (Methocel K4M Premium® | 112 |
| (c) Lactose B.P. | 53 |
| (d) Povidone B.P.C. | 28 |
| (e) Magnesium Stearate | 7 |
| | 700 |

Drug release took place over a period of about 6—8 hours and was complete after 12 hours.

Example 2 Capsule formulations:
Formulation A

A capable formulation was prepared by admixing the ingredients of Formulation D in Example 1 above and filling into a two-part hard gelatin capsule. Formulation B (*infra*) was prepared in a similar manner.

6

| Formulation B | mg/capsule |
|---|---|
| (a) Active ingredient | 250 |
| (b) Lactose B.P. | 143 |
| (c) Sodium Starch Glycollate | 25 |
| (d) Magnesium Stearate | 2 |
| | 420 |

| Formulation C | mg/capsule |
|---|---|
| (a) Active ingredient | 250 |
| (b) Macrogol 4000 BP® | 350 |
| | 600 |

Capsules were prepared by melting the Macrogol 4000 BP, dispersing the active ingredient in the melt and filling the melt into a two-part hard gelatin capsule.

| Formulation D | mg/capsule |
|---|---|
| Active Ingredient | 250 |
| Lecithin | 100 |
| Arachis Oil | 100 |
| | 450 |

Capsules were prepared by dispersing the active ingredient in the lecithin and arachis oil and filling the dispersion into soft, elastic gelatin capsules.

Formulation E (Controlled release capsule)

The following controlled release capsule formulation was prepared by extruding ingredients (a), (b) and (c) using an extruder, followed by spheronisation of the extrudate and drying. The dried pellets were then coated with release controlling membrane (d) and filled into a two-piece, hard gelatin capsule.

| | mg/capsule |
|---|---|
| (a) Active Ingredient | 250 |
| (b) Microcrystalline Cellulose | 125 |
| (c) Lactose BP | 125 |
| (d) Ethyl Cellulose | 13 |
| | 513 |

Example 3: Injectable formulation:

Formulation A

| | |
|---|---|
| Active ingredient | 0.200 g |
| Hydrochloric acid solution, 0.1M q.s. to | pH 4.0 to 7.0 |
| Sodium hydroxide solution, 0.1M q.s. to | pH 4.0 to 7.0 |
| Sterile water q.s. to | 10 ml |

The active ingredient was dissolved in most of the water (35°—40°C) and the pH adjusted to between 4.0 and 7.0 with the hydrochloric acid or the sodium hydroxide as appropriate. The batch was then made up to volume with the water and filtered through a sterile micropore filter into a sterile 10 ml amber glass vial (type 1) and sealed with sterile closures and overseals.

Formulation B

| | |
|---|---|
| Active ingredient | 0.125 g |
| Sterile, pyrogen-free, pH 7 phosphate buffer. q.s. to | 25 ml |

Example 4: Intramuscular injection:

| | |
|---|---|
| Active ingredient | 0.20 g |
| Benzyl Alcohol | 0.10 g |
| Glycofurol® 75 | 1.45 g |
| Water for Injection q.s. to | 3.00 ml |

The active ingredient was dissolved in the glycofurol. The benzyl alcohol was then added and dissolved, and water added to 3 ml. The mixture was then filtered through a sterile micropore filter and sealed in sterile 3 ml amber glass vials (type 1).

Example 5: Syrup:

| Active ingredient | 0.2500 g |
|---|---|
| Sorbitol Solution | 1.5000 g |
| Glycerol | 2.0000 g |
| Sodium Benzoate | 0.0050 g |
| Flavour, Peach 17.42.3169 | 0.0125 ml |
| Purified Water q.s. to | 5.0000 ml |

The active ingredient was dissolved in a mixture of the glycerol and most of the purified water. An aqueous solution of the sodium benzoate was then added to the solution, followed by addition of the sorbitol solution and finally the flavour. The volume was made up with purified water and mixed well.

Example 6: Suppository:

| | mg/suppository |
|---|---|
| Active ingredient (63 μm)* | 250 |
| Hard Fat, BP (Witepsol H15®—Dynamit Nobel) | 1770 |
| | 2020 |

*The active ingredient was used as a powder wherein at least 90% of the particles were of 63 μm diameter or less.

One-fifth of the Witepsol H15® was melted in a steam-jacketed pan at 45°C maximum. The active ingredient was sifted through a 200 μm sieve and added to the molten base with mixing, using a silverson fitted with a cutting head, until a smooth dispersion was achieved. Maintaining the mixture at 45°C, the remaining Witepsol H15® was added to the suspension and stirred to ensure a homogenous mix. The entire suspension was passed through a 250 μm stainless steel screen and, with continuous stirring, was allowed to cool to 40°C. At a temperature of 38°C to 40°C 2.02 g of the mixture was filled into suitable plastic moulds. The suppositories were allowed to cool to room temperature.

Example 7: Pessaries:

| | mg/pessary |
|---|---|
| Active ingredient 63 μm | 250 |
| Anhydrate Dextrose | 380 |
| Potato Starch | 363 |
| Magnesium Stearate | 7 |
| | 1000 |

The above ingredients were mixed directly and pessaries prepared by direct compression of the resulting mixture.

Reference Example: 3'-Azido-3'-deoxythymidine
a) 2,3'-Anhydrothymidine
Thymidine (85.4 g: 0.353 mol) was dissolved in 500 ml dry DMF and added to N-(2-chloro-1,1,2-trifluoroethyl)diethylamine (100.3 g; 0.529 mol) (prepared according to the method of D. E. Ayer, J. Med. Chem. 6, 608 (1963)). This solution was heated at 70°C for 30 minutes then poured into 950 ml ethanol (EtOH) with vigorous stirring. The product precipitated from this solution and was filtered. The EtOH supernatant was refrigerated then filtered to yield the title compound. mp.=228—230°C.

b) 3'-Azido-3'-deoxythymidine
2,3'-0-Anhydrothymidine (25 g: 0.1115 mol) and $NaN_3$ (29 g, 0.446 mol) was suspended in a mixture of 250 ml DMF and 38 ml water. The reaction mixture was refluxed for 5 hours at which time it was poured into 1 liter of water. The aqueous solution was extracted with EtOAc (3×700 ml). The EtOAc extracts were dried over $Na_2SO_4$, filtered and the EtOAc was removed in vacuo to yield a viscous oil. This oil was stirred with 200 ml water providing the title comopund as a solid which was collected by filtration. mp=116—118°C.

8

Antiviral activity

(a) (i) Retrovirus—Induced malignancy

3'-Azido-3'-deoxythymidine was administered to female BALB/c mice infected with $1.5 \times 10^4$ Pfu of the RVB3 strain of Rauscher Murine Leukaemia Virus. Treatment was started 4 hours after infection at dosages of 80 mg/kg intraperitoneally every 8 hours or 0.5 or 1.0 mg/ml orally in drinking water. Such treatment was found to prevent infection of spleen cells and subsequent development of splenomegaly and also suppressed viraemia.

(ii) HTLV-I

TM-11 cells (T-cell clone susceptible to HTLV-I infection) were co-cultivated with irradiated, HTLV-I producer MJ-tumour cells as follows:

a) TM-11 cells only;
b) TM-11 cells and MJ-tumour cells;
c) TM-11 cells, MJ-tumour cells and 3'-azido-3'-deoxythymidine (3 µM);
d) TM-11 cells, MJ-tumour cells and 3'-azido-3'-deoxythymidine (9 µM);
e) TM-11 cells, MJ-tumour cells and 3'-azido-3'-deoxythymidine (27 µM).

On day 18, total DNA was extracted from each culture and digested with Bam H1 to generate a fragment of the HTLV-I genome, independent of any host flanking sequence and having a standard molecular weight of 3.3 kD. The digest was then probed with radio-labelled lambdha MT-2, a standard probe recognising the Bam H1 fragment of HTLV-I.

No hybridisation was observed for a), indicating a lack of virus in the uninfected control. A strong signal was seen for b), the untreated, infected control. A weak signal was observed with c), indicating incomplete eradication of the virus, and no hybridisation was noted in d) or e) indicating complete extermination of the virus.

Each culture was also probed with a probe for T-cell receptor β chain, with a strong signal being generated for all cultures, showing the continued presence of TM-11 for the duration of the experiment.

(b) AIDV
(i) Reverse transcriptase activity

3'-Azido-5'-triphosphate-3'-deoxythymidine was tested *in vitro* against AIDV reverse transcriptase (AIDV RT).

AIDV RT was purified from pelleted and extracted AIDV by elution through DEAE and phosphocellulose columns. The enzyme activity was linear through 60 minutes and stable for at least 2 months when stored in 60% glycerol and 1 mg bovine serum albumin per ml. Using rA-odT$_{(12-18)}$ as the template-primer, AIDV RT had a pH optimum of 7.0 to 7.3, a MnCl$_2$ optimum of 0.3 mM and a MgCl$_2$ optimum of 5 mM. The activity in the presence of 5 mM MgCl$_2$ was 10-fold greater than the activity in the presence of 0.3 mM MnCl$_2$. Maximal enzyme activity was also found in 80 to 140 mM KCl and 60 to 100 mM NaCl. Incorporation of [$^3$H] dTTP was linear with respect to enzyme concentration. When tested, 3'-azido-5'-triphosphate-3'-deoxythymidine was found to be a competitive inhibitor of AIDV RT, giving a Ki of 0.04 µM when using rA-odT$_{(12-18)}$ as the template-primer. The enzyme had a Km for dTTP of 2.81 µM, suggesting that 3'-azido-5'-triphosphate-3'-deoxythymidine binds tighter to the enzyme than does dTTP. Further experiments with the RT's of avian myeloblastosis virus, Moloney murine leukemia virus and AIDV, showed 3'-azido-5'-triphosphate-3'-deoxythymidine to be a terminator of DNA chain elongation.

(ii) In vitro anti-AIDV activity

3'-Azido-3'-deoxythymidine was tested and found to possess activity in a number of *in vitro* assay systems. Drug effects were measured by assaying reverse transcriptase (RT) activity in the supernates from infected, uninfected, and drug treated cells, 3'-Azido-3'-deoxythymidine effectively blocked the infection by AIDV of the H9 and U937 human lymphoblastoid cell lines at concentrations from 2.7 to 0.0013 µg/ml. Similarly, infection of normal PHA stimulated white blood cells and cultured peripheral blood lymphocytes was inhibited at drug concentrations as low as 0.013 µg/ml. Drug addition and subtraction experiments in H9 cells revealed that 3'-azido-3'-deoxythymidine was most effective when present at the time of virus infection of susceptible cells, but still retained most of its antiviral activity even when added as late as 20 hours after initial AIDV infection. Inhibition of viral replication was also evident when the drug was present in the media only during the 20 hour period of virus absorption. Effects were seen at 0.13 and 0.013 µg/ml. 3'-Azido-3'-deoxythymidine exhibited no direct anti-RT activity against purified AIDV virions. Similarly, the drug had little or no effect on the production and release of virions from the chronically infected H9 AIDV cell line.

(iii) Preventing infection by AIDV

The ability of 3'-azido-3'-deoxythymidine to block infection of cells by AIDV was determined as follows. Cloned T4 positive tetanus specific T helper lymphocytes were infected with a pool of AIDV isolates [at challenge doses of up to 5000 virions/cell] and cell survival after infection was monitored. After 10 days in culture no viral cytopathic effects were seen in infected T cells treated with 8.8 and 1.3 µg/ml 3'-azido-3'-deoxythymidine, while untreated, infected cells were 5-fold decreased. Cell survival was also evaluated in an HTLV-I transformed, AIDV superinfected cell line derived from the cells above. 3'-azido-3'-deoxythymidine at concentrations of 2.7, 0.27 and 0.13 µg/ml totally blocked cytopathic effects at 7 days. Protective effects were seen in infections induced by both cell free virions and cell associated virus. 3'-Azido-3'-deoxythymidine at 0.27 µg/ml concentration also effectively prevented cytopathic effect induction by a less related Haitian isolate of AIDV.

Toxicity assay

3'-Azido-3'-deoxythymidine was administered to both mice and rats. The $LD_{50}$ value was in excess of 750 mg/kg in both species.

**Claims for the Contracting States: BE, CH, DE, FR, GB, IT, LI, LU, NL, SE**

1. A pharmaceutical formulation comprising as active ingredient, 3'-azido-3'-deoxythymidine, and a at least one pharmaceutically acceptable carrier therefor.
2. A formulation according to claim 1 wherein the carrier is other than water.
3. A formulation according to claim 1 or 2 which is sterile.
4. A formulation according to claim 3 adapted for administration by injection.
5. A formulation according to claim 4 contained in a sealed vial.
6. A formulation according to either of claim 3 and 4 wherein the carrier is sterile water.
7. A formulation according to claim 1 or 2 adapted for oral administration.
8. A formulation according to any of claims 1, 2 or 7 in the form of a tablet or capsule.
9. A formulation according to any of claims 1, 2 or 3 providing sustained release of the active ingredient after oral administration.
10. A formulation according to claim 7 further comprising a flavouring agent.
11. A formulation according to any of claims 1 to 10 comprising a unit dose of the active ingredient, or a multiple thereof.
12. A formulation according to claim 11 wherein the unit dose is between 5 and 1500 mg of the active ingredient.
13. A formulation according to claim 11 wherein the unit dose is between 10 and 1000 mg of the active ingredient.
14. A formulation according to claim 11 wherein the unit dose is between 20 and 700 mg of the active ingredient.
15. A process for the preparation of a formulation according to any of claims 1 to 14 which comprises bringing 3'-azido-3'-deoxythymidine into association with at least one pharmaceutically acceptable carrier therefor.

**Claims for the Contracting State: AT**

1. A process for the preparation of a pharmaceutical formulation comprising bringing 3'-azido-3'-deoxythymidine as active ingredient, into association with at least one a pharmaceutically acceptable carrier therefor.
2. A process for the preparation of a formulation according to claim 1 wherein the carrier is other than water.
3. A process for the preparation of a formulation according to claim 1 or 2 wherein the ingredients are sterile.
4. A process for the preparation of a formulation according to claim 3 wherein said formulation is adapted for administration by injection.
5. A process for the preparation of a formulation according to claim 4 further comprising storing in a sealed vial.
6. A process for the preparation of a formulation according to either of claims 3 and 4 wherein the carrier is sterile water.
7. A process for the preparation of a formulation according to claim 1 or 2 wherein said formulation is further adapted for oral administration.
8. A process for the preparation of a formulation according to claim 1, 2 or 7 wherein said formulation is made in the form of a tablet or capsule.
9. A process for the preparation of a formulation according to claim 1, 2 or 3 wherein said formulation is further adapted to provide sustained release of the active ingredient after oral administration.
10. A process for the preparation of a formulation according to claim 7 further comprising the addition of a flavouring agent.
11. A process for the preparation of a formulation according to any of claims 1 to 10 wherein the active ingredient is present as a unit dose, or a multiple thereof.
12. A process for the preparation of a formulation according to claim 11 using between 5 and 1500 mg of the active ingredient.
13. A process for the preparation of a formulation according to claim 11 using between 10 and 1000 mg of the active ingredient.

14. A process for the preparation of a formulation according to claim 11 using between 20 and 700 mg of the active ingredient.

## Patentansprüche für die Vertragsstaaten: BE, CH, DE, FR, GB, IT, LI, LU, NL, SE

1. Pharmazeutische Formulierung, enthaltend als aktiven Bestandteil 3'-Azido-3'-desoxythymidin und mindestens einen pharmazeutisch verträglichen Träger dafür.

2. Formulierung nach Anspruch 1, worin der Träger von Wasser verschieden ist.

3. Formulierung nach Anspruch 1 oder 2, die steril ist.

4. Formulierung nach Anspruch 3, adaptiert für die Verabreichung durch Injektion.

5. Formulierung nach Anspruch 4, die in einem hermetisch verschlossenen Fläschchen enthalten ist.

6. Formulierung nach einem der Ansrpüche 3 und 4, worin der Träger steriles Wasser ist.

7. Formulierung nach Anspruch 1 oder 2, die für eine orale Verabreichung adaptiert ist.

8. Formulierung nach Anspruch 1, 2 oder 7 in Form einer Tablette oder einer Kapesel.

9. Formulierung nach Anspruch 1, 2 oder 3, die nach oraler Verabreichung eine langzeitwirkende Freisetzung des aktiven Bestandteils gewährleistet.

10. Formulierung nach Anspruch 7, die weiter einen Geschmacksstoff enthält.

11. Formulierung nach einem der Ansprüche 1 bis 10, enthaltend eine Einzeldosis oder eine Mehrfachdosis des aktiven Bestandteils.

12. Formulierung nach Anspruch 11, worin die Einzeldosis zwischen 5 und 1500 mg des aktiven Bestandteils beträgt.

13. Formulierung nach Anspruch 11, worin die Einzeldosis zwischen 10 und 1000 mg des aktiven Bestandteils beträgt.

14. Formulierung nach Anspruch 11, worin die Einzeldosis zwischen 20 und 700 mg des aktiven Bestandteils beträgt.

15. Verfahren zur Herstellung einer Formulierung nach einem der Ansprüche 1 bis 14, bei dem man 3'-Azido-3'-desoxythymidin mit mindestens einem pharmazeutische verträglichen Träger dafür vereinigt.

## Patentansprüche für den Vertragsstaat: AT

1. Verfahren zur Herstellung einer pharmazeutischen Formulierung, bei dem man 3'-Azido-3'-desoxythymidin als aktiven Bestandteil mit mindestens einem pharmazeutisch verträglichem Träger dafür vereinigt.

2. Verfahren zur Herstellung einer Formulierung nach Anspruch 1, bei dem der Träger von Wasser verschieden ist.

3. Verfahren zur Herstellung einer Formulierung nach Anspruch 1 oder 2, bei dem die Bestandteile steril sind.

4. Verfahren zur Herstellung einer Formulierung nach Anspruch 3, bei dem die Formulierung für die Verabreichung durch Injektion adaptiert wird.

5. Verfahren zur Herstellung einer Formulierung nach Anspruch 4, zusätzlich umfassend die Aufbewahrung in einem hermetisch verschlossenen Flächchen.

6. Verfahren zur Herstellung einer Formulierung nach einem der Ansprüche 3 und 4, bei dem der Träger steriles Wasser ist.

7. Verfahren zur Herstellung einer Formulierung nach Anspruch 1 oder 2, bei dem die Formulierung weiterhin für eine orale Verabreichung adaptiert wird.

8. Verfahren zur Herstellung einer Formulierung nach Anspruch 1, 2 oder 7, bei·dem die Formulierung in Form einer Tablette oder einer Kapsel hergestellt wird.

9. Verfahren zur Herstellung einer Formulierung nach Anspruch 1, 2 oder 3, bei dem die Formulierung weiterhin für eine langzeitwirkende Freisetzung des aktiven Bestandteils nach oraler Verabreichung adaptiert wird.

10. Verfahren zur Herstellung einer Formulierung nach Anspruch 7, bei dem zusätzlich ein Geschmacksstoff beigegeben wird.

11. Verfahren zur Herstellung einer Formulierung nach einem der Ansprüche 1 bis 10, bei dem der aktive Bestandteil als Einzeldosis oder als Mehrfachdosis vorliegt.

12. Verfahren zur Herstellung einer Formulierung nach Anspruch 11, bei dem man zwischen 5 und 1500 mg des aktiven Bestandteils verwendet.

13. Verfahren zur Herstellung einer Formulierung nach Anspruch 11, bei dem man zwischen 10 und 1000 mg des aktiven Bestandteils verwendet.

14. Verfahren zur Herstellung einer Formulierung nach Anspruch 11, bei dem man zwischen 20 und 700 mg des aktiven Bestandteils verwendet.

## Revendications pour les Etats Contractants: BE, CH, DE, FR, GB, IT, LI, LU, NL, SE

1. Composition pharmaceutique, comprenant comme constituant actif, de la 3'-azido-3'-désoxythymidine et au moins un excipient pharmaceutiquement acceptable pour celle-ci.

# EP 0 196 185 B1

2. Composition suivant la revendication 1, dans laquelle l'excipient est autre que de l'eau.

3. Composition suivant la revendication 1 ou 2, qui est stérile.

4. Composition suivant la revendication 3, adaptée à l'administration par injection.

5. Composition suivant la revendication 4, contenue dans une fiole scellée.

6. Composition suivant l'une quelconque des revendications 3 et 4, dans laquelle l'excipient est de l'eau stérile.

7. Composition suivant la revendication 1 ou 2, adaptée à l'administration par voie orale.

8. Composition suivant l'une quelconque des revendications 1, 2 et 7, sous la forme d'un comprimé ou d'une capsule.

9. Composition suivant l'une quelconque des revendications 1, 2 et 3, assurant le dégagement étalé du constituant actif après administration par voie orale.

10. Composition suivant la revendication 7, contenant, en outre, un agent aromatisant.

11. Composition suivant l'une quelconque des revendications 1 à 10, comprenant une dose unitaire du constituant actif, ou un multiple de celle-ci.

12. Composition suivant la revendication 11, dans laquelle la dose unitaire se situe entre 5 et 1500 mg du constituant actif.

13. Composition suivant la revendication 11, dans laquelle la dose unitaire se situe entre 10 et 1000 mg du constituant actif.

14. Composition suivant la revendication 11, dans laquelle la dose unitaire se situe entre 20 et 700 mg du constituant actif.

15. Procédé de préparation d'une composition suivant l'une quelconque des revendications 1 à 14, qui comprend la mise en association de la 3'-azido-3'-désoxythymidine avec au moins un excipient pharmaceutiquement acceptable pour celle-ci.

**Revendications pour l'Etat Contractant: AT**

1. Procédé de préparation d'une composition pharmaceutique, qui comprend la mise en association de la 3'-azido-3'-désoxythymidine comme constituant actif avec au moins un excipient pharmaceutiquèment acceptable pour celle-ci.

2. Procédé de préparation d'une composition suivant la revendication 1, dans lequel l'excipient est autre que de l'eau.

3. Procédé de préparation d'une composition suivant la revendication 1 ou 2, dans lequel les constituants sont stériles.

4. Procédé de préparation d'une composition suivant la revendication 3, dans lequel la composition est adaptée à l'administration par injection.

5. Procédé de préparation d'une composition suivant la revendication 4, qui comprend, en outre, la conservation dans une ampoule scellée.

6. Procédé de préparation d'une composition suivant la revendication 3 ou 4, dans lequel l'excipient est l'eau stérile.

7. Procédé de préparation d'une composition suivant la revendication 1 ou 2, dans lequel la composition est, en outre, adaptée à l'administration par voie orale.

8. Procédé de préparation d'une composition suivant la revendication 1, 2 ou 7, dans lequel la composition est préparée sous la forme d'un comprimé ou d'une capsule.

9. Procédé de préparation d'une composition suivant la revendication 1, 2 ou 3, dans lequel la composition est, en outre, adaptée pour assurer le dégagement étalé du constituant actif après administration par voie orale.

10. Procédé de préparation d'une composition suivant la revendication 7, qui comprend, en outre, l'addition d'un agent aromatisant.

11. Procédé de préparation d'une composition suivant l'une quelconque des revendications 1 à 10, dans lequel le constituant actif est présent à l'état de dose unitaire ou de multiple de celle-ci.

12. Procédé de préparation d'une composition suivant la revendication 11, en utilisant entre 5 et 1500 mg du constituant actif.

13. Procédé de préparation d'une composition suivant la revendication 11, en utilisant entre 10 et 1000 mg du constituant actif.

14. Procédé de préparation d'une composition suivant la revendication 11, en utilisant entre 20 et 700 mg du constituant actif.

12